# EUROPEAN PATENT APPLICATION

(11) **EP 0 576 898 A2**
(43) Date of publication of application: **05.01.1994**
(21) Application number: 93109532.7
(22) Date of filing: 15.06.1993
(51) Int. Cl.: C07K 7/06, C07K 7/08, A61K 37/02

(54) **Linear peptides**

(30) Priority: 18.06.1992 US 900385
(71) Applicant: MERCK PATENT GmbH, D-64271 Darmstadt (DE)
(72) Inventor: Jonczyk, Alfred, Dr., D-64295 Darmstadt (DE); Felding-Habermann, Brundhilde, Dr., La Jolla, CA 92037 (US); Melzer, Guido, Dr., D-65719 Hofheim/Ts. (DE); Diefenbach, Beate, D-64287 Darmstadt (DE); Cheresh, David A., Dr., La Jolla, Ca 92037 (US)

(57) **Abstract**

The invention relates to novel linear peptides of Formulae Ia - In
(Ia) H-Leu-Leu-Val-Gly-Ala-Pro-Leu-Tyr-OH;
(Ib) H-Asp-Leu-Leu-Val-Gly-Ala-Pro-Leu-Tyr-OH;
(Ic) H-His-Asp-Leu-Leu-Val-Gly-Ala-Pro-Leu-Tyr-OH;
(Id) H-Arg-His-Asp-Leu-Leu-Val-Gly-Ala-Pro-Leu-Tyr-OH;
(Ie) H-Gly-Arg-His-Asp-Leu-Leu-Val-Gly-Ala-Pro-Leu-Tyr-OH;
(If) H-Asp-Gly-Arg-His-Asp-Leu-Leu-Val-Gly-Ala-Pro-Leu-Tyr-OH;
(Ig) H-Gly-Asp-Gly-Arg-His-Asp-Leu-Leu-Val-Gly-Ala-Pro-Leu-Tyr-OH;
(Ih) H-Gly-Asp-Gly-Arg-His-Asp-Leu-OH;
(Ii) H-Gly-Asp-Gly-Arg-His-Asp-Leu-Leu-OH;
(Ij) H-Gly-Asp-Gly-Arg-His-Asp-Leu-Leu-Val-Gly-OH;
(Ik) H-Gly-Asp-Gly-Arg-His-Asp-Leu-Leu-Val-Gly-Ala-OH;
(Il) H-Gly-Asp-Gly-Arg-His-Asp-Leu-Leu-Val-Gly-Ala-Pro-Leu-OH;
(Im) Ac-His-Asp-Leu-Leu-Val-Gly-Ala-Pro-Leu-OH;
(In) Ac-Gly-Arg-His-Asp-Leu-Leu-Val-Gly-Ala-Pro-Leu-OH;
wherein Ac stands for acetyl, and salts thereof.

These substances inhibit, inter alia, cell adhesion.

## Description

### BACKGROUND OF THE INVENTION

This invention relates to new linear peptides having valuable properties.
Similar, but cyclic compounds are known from European Patent Application No. 0,406,428.

### Summary of Invention

It is an object of the invention to provide new compounds with useful, properties, in particular those which can be used for the preparation of medicaments.

Upon further study of the specification and appended claims, further objects and advantages of this invention will become apparent to those skilled in the art.

These objects have been achieved by providing new linear peptides of the formulae Ia - In:
(Ia) H-Leu-Leu-Val-Gly-Ala-Pro-Leu-Tyr-OH;
(Ib) H-Asp-Leu-Leu-Val-Gly-Ala-Pro-Leu-Tyr-OH;
(Ic) H-His-Asp-Leu-Leu-Val-Gly-Ala-Pro-Leu-Tyr-OH;
(Id) H-Arg-His-Asp-Leu-Leu-Val-Gly-Ala-Pro-Leu-Tyr-OH;
(Ie) H-Gly-Arg-His-Asp-Leu-Leu-Val-Gly-Ala-Pro-Leu-Tyr-OH;
(If) H-Asp-Gly-Arg-His-Asp-Leu-Leu-Val-Gly-Ala-Pro-Leu-Tyr-OH;
(Ig) H-Gly-Asp-Gly-Arg-His-Asp-Leu-Leu-Val-Gly-Ala-Pro-Leu-Tyr-OH;
(Ih) H-Gly-Asp-Gly-Arg-His-Asp-Leu-OH;
(Ii) H-Gly-Asp-Gly-Arg-His-Asp-Leu-Leu-OH;
(Ij) H-Gly-Asp-Gly-Arg-His-Asp-Leu-Leu-Val-Gly-OH;
(Ik) H-Gly-Asp-Gly-Arg-His-Asp-Leu-Leu-Val-Gly-Ala-OH;
(Il) H-Gly-Asp-Gly-Arg-His-Asp-Leu-Leu-Val-Gly-Ala-Pro-Leu-OH;
(Im) Ac-His-Asp-Leu-Leu-Val-Gly-Ala-Pro-Leu-OH;
(In) Ac-Gly-Arg-His-Asp-Leu-Leu-Val-Gly-Ala-Pro-Leu-OH;
wherein Ac stands for acetyl and salts thereof.

It has been found that the compounds of the formulae Ia-In and their salts have very useful properties. In particular, they are inhibitors of cell adhesion, useful to inhibit, e.g., the aggregation of blood-cells and tumor-cells. Thus, the compounds can be used to inhibit adhesion in animal cells, for example, somatic cells or cancer cells of mammals.

These inhibition effects can be detected, e.g,. by the methods which are given in EP 0,406,428, FEBS Lett., 291, 50-54 (1991) or by Smith, J.W., Ruggeri, Z.M., Kunicki, T.J., Cheresh, D.A., J. Biol. Chem., 265, 12267-12271 (1990).

The compounds can be employed as pharmaceutically active compounds in human and veterinary medicine, in particular for the treatment and prophylaxis of thrombosis, myocardial infarct, angina pectoris, apoplexy and for tumors, that means cancer diseases.

The abbreviations of amino acid radicals shown above and below stand for the radicals of the following amino acids:
- Ala: Alanine
- Arg: Arginine
- Asp: Aspartic acid
- Gly: Glycine
- His: Histidine
- Leu: Leucine
- Pro: Proline
- Tyr: Tyrosine
- Val: Valine.

In addition, the following have the meanings below:
- Ac: acetyl
- BOC: tert.-butoxycarbonyl
- CBZ: benzyloxycarbonyl
- DCCI: dicyclohexylcarbodiimide
- DMF: dimethylformamide
- EDCI: N-ethyl-N'-(3-dimethylaminopropyl)carbodiimidehydrochloride
- FMOC: 9-fluorenylmethoxycarbonyl
- Me: methyl
- OMe: methoxy
- OEt: ethoxy
- TFA: trifluoroacetic acid.
- POA: phenoxyacetyl
If individual compounds are mentioned above and below, then the abbreviations of these amino acids in each case relate to the L-form, if not expressly stated otherwise.

The invention further relates to a process for the preparation of a linear peptide of the formula I, characterized in that it is liberated from one of its functional derivatives by treating with a solvolyzing or hydrogenolyzing agent, or in that two peptides with smaller sequences are combined by peptide fragment condensation to give one of the peptides of formula I and/or a compound of formula I is converted into one of its salts by treating with an acid.

The compounds of the formula I and also the starting materials for their preparation are otherwise prepared by known methods, as are described in the literature (e.g., in the standard works such as Houben-Weyl. Methoden der organischen Chemie, (Methods of Organic Chemistry) Georg-Thieme-Verlag, Stuttgart), in particular under reaction conditions which are known and suitable for the said reactions. In this context, use can also be made of known variants which are not mentioned in more detail here.

The starting substances can also be formed in situ, if desired, such that they are not isolated from the reaction mixture, but immediately reacted further to give the compounds of the formula I.

The compounds of the formula I can be obtained by liberating them from their functional derivatives by solvolysis, in particular hydrolysis, or by hydrogenolysis.

Preferred starting materials for the solvolysis or hydrogenolysis are those which contain appropriate protected amino and/or hydroxyl groups instead of one or more free amino and/or hydroxyl groups, preferably those which carry an amino protecting group instead of an H atom which is bonded to an N atom, e.g., those which correspond to the formula I, but contain an NHR' group (in which R' is an amino protecting group, e.g., BOC or CBZ) instead of an NH₂ group.

In addition, starting materials are preferred which carry a hydroxyl protecting group instead of the H atom of a hydroxyl group, e.g., those which correspond to the formula I, but contain an R''O-phenyl group (in which R'' is a hydroxyl protecting group) instead of a hydroxyphenyl group.

Several - identical or different - protected amino and/or hydroxyl groups can be present in the molecule of the starting material. If the protective groups present are different from one another, in many cases they can be removed selectively.

The expression "amino protecting group" is generally known and relates to groups which are suitable for protecting (for blocking) an amino group from chemical reactions, but which are easily removable, after the desired chemical reaction has been carried out on other positions of the molecule. Typical groups of this type are, in particular, unsubstituted or substituted acyl, aryl, aralkoxymethyl or aralkyl groups. As the amino protecting groups are removed after the desired reaction (or reaction sequence), their nature and size is otherwise not critical; but those having 1-20, in particular 1-8, C atoms are preferred. The expression "acyl group" is to be taken in its widest sense in connection with the present process. It includes acyl groups derived from aliphatic, araliphatic, aromatic or heterocyclic carboxylic acids or sulfonic acids and in particular alkoxycarbonyl, aryloxycarbonyl and, above all, aralkoxycarbonyl groups. Examples of acyl groups of this type are alkanoyl such as acetyl, propionyl or butyryl; aralkanoyl such as phenylacetyl; aroyl such as benzoyl or toluyl; aryloxyalkanoyl such as POA:; alkoxycarbonyl such as methoxycarbonyl, ethoxycarbonyl, 2,2,2-trichloroethoxycarbonyl, BOC, 2-iodoethoxycarbonyl; aralkyloxycarbonyl such as CBZ ("carbobenzoxy"), 4-methoxybenzyloxycarbonyl and FMOC. Preferred amino protecting groups are BOC, and in addition CBZ, FMOC, benzyl and acetyl.

The expression "hydroxy protecting group" is also generally known and relates to groups which are suitable for protecting a hydroxyl group from chemical reactions, but which are easily removable, after the desired chemical reaction has been carried out on other positions of the molecule. Typical groups of this type are the above mentioned unsubstituted or substituted aryl, aralkyl or acyl groups, and in addition also alkyl groups. The nature and size of the hydroxy protecting groups is not critical, as they are removed again after the desired chemical reaction or reaction sequence; preferred groups are those having 1-20, in particular 1-10 C atoms. Examples of hydroxyl protecting groups are, inter alia, benzyl, p-nitrobenzoyl, tert.-butyl, p-toluenesulfonyl and acetyl, with benzyl, tert.-butyl and acetyl being particularly preferred.

The functional derivatives of the compounds of the formula I to be used as starting materials can be prepared by customary methods of amino acid and peptide synthesis, such as are described,e.g.,in the standard works and patent applications mentioned, and,e.g., also by the Merrifield solid phase method (B.F. Gysin and R.B. Merrifield, J. Am. Chem. Soc. 94, 3102 et seq. (1972)).

The liberation of the compounds of the formula I from their functional derivatives is carried out - depending on the protecting group used - e.g., with strong acids, preferably with TFA or perchloric acid, but also with other strong inorganic acids such as hydrochloric acid or sulfuric acid, or strong organic carboxylic acids such as trichloroacetic acid or sulfonic acids such as benzene-or p-toluenesulfonic acid. The presence of an additional inert solvent is possible, but not always necessary. Suitable inert solvents are preferably organic, for example carboxylic acids such as acetic acid, ethers such as tetrahydrofuran or dioxane, amides such as DMF, halogenated hydrocarbons such as dichloromethane, and in addition also alcohols such as methanol, ethanol or isopropanol and also water.

In addition, mixtures of the above mentioned solvents are suitable. TFA is preferably used in an excess without addition of a further solvent; perchloric acid in the form of a mixture of acetic acid and 70% perchloric acid in the ratio 9:1. The reaction temperatures for the cleavage are preferably between about 0 and about 50°, preferably between 15 and 30° (room temperature).

The BOC group can be removed, e.g., preferably using 40% TFA in dichloromethane or with about 3 to 5 N HCl in dioxane at 15-30°, the FMOC group using an about 5 to 20% solution of dimethylamine, diethylamine or piperidine in DMF at 15-30°.

Protecting groups which can be removed by hydrogenolysis (e.g., CBZ or benzyl) can be removed, e.g., by treating with hydrogen in the presence of a catalyst (e.g., a precious metal catalyst such as palladium, preferably on a carrier such as carbon). Suitable solvents in this case are those mentioned above, in particular, e.g., alcohols such as methanol or ethanol or amides such as DMF. The hydrogenolysis is carried out, as a rule, at temperatures between about 0 and 100° and pressures between about 1 and 200 bar, preferably at 20-30° and 1-10 bar. Hydrogenolysis of the CBZ group is easily carried out, e.g., on 5 to 10% Pd-C in methanol or using ammonium formate (instead of Hz) on Pd-C in methanol/DMF at 20-30°.

Compounds of the formula I can also be obtained by condensation of peptides with smaller sequences under the conditions of a peptide synthesis. In this case, the reaction is preferably carried out by customary methods of peptide synthesis, as are described, e.g., in Houben-Weyl, loc cit. volume 15/II, pages 1 to 806 (1974).

The reaction is preferably carried out in the presence of a dehydrating agent, e.g., a carbodiimide such as DCCI or EDCI, and in addition propanephosphonic anhydride (compare Angew. Chem 92, 129 (1989)), diphenylphosphoryl azide or 2-ethoxy-N-ethoxycarbonyl-1,2-dihydroquinoline, in an inert solvent, e.g., a halogenated hydrocarbon such as dichloromethane, an ether such as tetrahydrofuran or dioxane, an amide such as DMF or dimethylacetamide, a nitrile such as acetonitrile, or in mixtures of these solvents, at temperatures between about -10 and 40, preferably between 0 and 30°.

A base of the formula I can be converted into the appropriate acid addition salt using an acid. Suitable acids for this reaction are in particular those which yield physiologically acceptable salts. Inorganic acids can thus be used, e.g., sulfuric acid, nitric acid, hydrohalic acids such as hydrochloric acid or hydrobromic acid, phosphoric acid such as orthophosphoric acid and sulfamic acid, and in addition organic acids, in particular aliphatic, alicyclic, araliphatic, aromatic or heterocyclic mono- or polybasic carboxylic, sulfonic or sulfuric acids, e.g., formic acid, acetic acid, propionic acid, pivalic acid, diethylacetic acid, malonic acid, succinic acid, pimelic acid, fumaric acid, maleic acid, lactic acid, tartaric acid, malic acid, benzoic acid, salicylic acid, 2- or 3-phenylpropionic acid, citric acid, gluconic acid, ascorbic acid, nicotinic acid, isonicotinic acid, methane- or ethanesulfonic acid, ethanedisulfonic acid, 2-hydroxyethanesulfonic acid, benzenesulfonic acid, p-toluenesulfonic acid, naphthalene-mono- and -disulfonic acids, and laurylsulfuric acid. Salts with physiologically unacceptable acids, e.g., picrates, can be used for the isolation and/or purification of the compounds of the formula I.

The novel compounds of the formula I and their physiologically acceptable salts can be used for the production of pharmaceutical preparations by bringing them into a suitable dosage form together with at least one excipient or auxiliary and, if desired, together with one or more other active compound(s). The preparations thus obtained can be employed as pharmaceuticals in human or veterinary medicine. Suitable excipient substances are organic or inorganic substances which are suitable for enteral (e.g., oral or rectal), parenteral or local (e.g., topical) administration or for administration in the form of an inhalant spray and which do not react with the novel compounds, for example water, lower alcohols vegetable oils, benzyl alcohols, polyethylene glycols, glycerol triacetate and other fatty acid glycerides, gelatin, soya lecithin, carbohydrates such as lactose or starch, magnesium stearate, talc, cellulose and vaseline. Tablets, coated tablets, capsules, syrups, liquids or drops, in particular, are used for oral administration; film tablets and capsules having gastric juice-resistant coatings or capsule shells are especially of interest. Suppositories are used for rectal administration, and solutions, preferably oily or aqueous solutions, and in addition suspensions, emulsions or implants, are used for parenteral administration. Solutions, e.g., which can be used in the form of eye drops, and in addition, e.g., suspensions, emulsions, creams, ointments or compresses are suitable for topical application. Sprays can be used which contain the active compound either dissolved or suspended in a propellant gas mixture (e.g., chlorofluorohydrocarbons or a ecologically harmless substitute) for administration as inhalant sprays. The active compound here is preferably used in micronized form, it being possible for one or more additional physiologically tolerable solvents to be present, e.g. ethanol. Inhalant solutions can be administered with the aid of customary inhalers. The novel compounds can also be lyophylized and the lyophylizates obtained used, e.g., for the production of injection preparations. The preparations indicated can be sterilized and/or can contain auxiliaries such as preservatives, stabilizers and/or wetting agents, emulsifiers, salts for influencing osmotic pressure, buffer substances, colorants and/or flavorings. If desired, they can also contain one or more other active compounds, e.g., one or more vitamins.

The substances according to the invention are as a rule administered in analogy to other known commercially available peptides, but in particular in analogy to the compounds described in US-A-4,472,305, preferably in dosages between about 0.05 and 500 mg, in particular between 0.5 and 100 mg per dosage unit. The daily dose is preferably between about 0.01 and 2 mg/kg of body weight. The specific dose for each intended patient depends however, on many different factors, for example the activity of the specific compound employed, the age, body weight, general state of health, sex, the cost, the time and route of administration, and the rate of excretion, pharmaceutical combination and severity of the particular disorder to which the therapy applies. Parenteral administration is preferred.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the foregoing and in the following examples, all temperatures are set forth uncorrected in degrees Celsius and unless otherwise indicated, all parts and percentages are by weight.

The entire disclosure of all applications, patents and publications, cited above and below are hereby incorporated by reference.

In the following examples, "customary working up" means: water is added if necessary, the mixture is neutralized and extracted with ether or dichloromethane, the organic phase is separated off, dried over sodium sulfate, filtered and evaporated and the residue is purified by chromatography on silica gel and/or crystallization. RT = retention time (minutes) for HPLC on an Lichrosorb® RP Select B (250-4.7µm) column, if not stated otherwise; eluent: a = 0.3% TFA in water, b = isopropanol (gradient 0-80 vol.-%).

The molecule mass of the compounds was determined by fast atom bombardment (FAB; M⁺: molion peak).

### EXAMPLES

### Example 1

2,0 g of BOC-Leu-Leu-Val-OH [obtainable, e.g., by modified Merrifield techniques for peptide synthesis] are dissolved in a mixture of 150 ml of dichloromethane and 20 ml of DMF and cooled to 0°, 0.5 g of DCCI, 0.3 g of HOBt, 0.23 ml of N-methyl- morpholine and 1 equivalent of H-Gly-Ala-Pro-Leu-Tyr-OMe are added successively with stirring, and the mixture is stirred for a further 24 hours at 0° and 12 hours at 25°. Afterwards the solution is concentrated and treated with a mixed bed ion exchanger to free it from salts. This is then filtered off, the solution is evaporated and the residue is purified. BOC-Leu-Leu-Val-Gly-Ala-Pro-Leu-Tyr-OMe is obtained.

The following are obtained analogously:

BOC-Asp-Leu-Leu-Val-Gly-Ala-Pro-Leu-Tyr-OMe;
BOC-His-Asp-Leu-Leu-Val-Gly-Ala-Pro-Leu-Tyr-OMe;
BOC-Arg-His-Asp-Leu-Leu-Val-Gly-Ala-Pro-Leu-Tyr-OMe;
BOC-Gly-Arg-His-Asp-Leu-Leu-Val-Gly-Ala-Pro-Leu-Tyr-OMe;
BOC-Asp-Gly-Arg-His-Asp-Leu-Leu-Val-Gly-Ala-Pro-Leu-Tyr-OMe;
BOC-Gly-Asp-Gly-Arg-His-Asp-Leu-Leu-Val-Gly-Ala-Pro-Leu-Tyr-OMe;
BOC-Gly-Asp-Gly-Arg-His-Asp-Leu-OMe;
BOC-Gly-Asp-Gly-Arg-His-Asp-Leu-Leu-OMe;
BOC-Gly-Asp-Gly-Arg-His-Asp-Leu-Leu-Val-Gly-OMe;
BOC-Gly-Asp-Gly-Arg-His-Asp-Leu-Leu-Val-Gly-Ala-OMe;
BOC-Gly-Asp-Gly-Arg-His-Asp-Leu-Leu-Val-Gly-Ala-Pro-Leu-OMe;
BOC-His-Asp-Leu-Leu-Val-Gly-Ala-Pro-Leu-OMe;
BOC-Gly-Arg-His-Asp-Leu-Leu-Val-Gly-Ala-Pro-Leu-OMe.

### Example 2

1.8 g of BOC-Leu-Leu-Val-Gly-Ala-Pro-Leu-Tyr-OMe are dissolved in 60 ml of methanol, 1.5 ml of 2 N sodium hydroxide solution are added and the mixture is stirred for 3 hours at 20°. After evaporation the residue is taken up in water, acidified to pH 3 with dilute HCl and extracted with ethyl acetate. The extract is dried over Na₂SO₄, evaporated again and the BOC-Leu-Leu-Val-Gly-Ala-Pro-Leu-Tyr-OH obtained is stirred at 20° for 2 hours with 20 ml of 2 N HCl in dioxane.

Subsequently the mixture is evaporated and the residue is purified by HPLC to give H-Leu-Leu-Val-Gly-Ala-Pro-Leu-Tyr-OH; M⁺ 846.

The following are obtained analogously:

H-Asp-Leu-Leu-Val-Gly-Ala-Pro-Leu-Tyr-OH; M+ 961;
H-His-Asp-Leu-Leu-Val-Gly-Ala-Pro-Leu-Tyr-OH; M+ 1098;
H-Arg-His-Asp-Leu-Leu-Val-Gly-Ala-Pro-Leu-Tyr-OH; M+ 1252;
H-Gly-Arg-His-Asp-Leu-Leu-Val-Gly-Ala-Pro-Leu-Tyr-OH; M+ 1309;
H-Asp-Gly-Arg-His-Asp-Leu-Leu-Val-Gly-Ala-Pro-Leu-Tyr-OH; M+ 1425;
H-Gly-Asp-Gly-Arg-His-Asp-Leu-Leu-Val-Gly-Ala-Pro-Leu-Tyr-OH; M+ 1482;
H-Gly-Asp-Gly-Arg-His-Asp-Leu-OH; M+ 769;
H-Gly-Asp-Gly-Arg-His-Asp-Leu-Leu-OH; M+ 882;
H-Gly-Asp-Gly-Arg-His-Asp-Leu-Leu-Val-Gly-OH; M+ 1038;
H-Gly-Asp-Gly-Arg-His-Asp-Leu-Leu-Val-Gly-Ala-OH; M+ 1109;
H-Gly-Asp-Gly-Arg-His-Asp-Leu-Leu-Val-Gly-Ala-Pro-Leu-OH; M+ 1319;
H-His-Asp-Leu-Leu-Val-Gly-Ala-Pro-Leu-OH; M+ 935;
H-Gly-Arg-His-Asp-Leu-Leu-Val-Gly-Ala-Pro-Leu-OH; M+ 1146.

### Example 3

2.7 g of H-His-Asp-Leu-Leu-Val-Gly-Ala-Pro-Leu-OH are dissolved in 200 ml of aqueous DMF and, while stirring, a solution of 1.0 g of acetyl chloride in 10 ml of dichloromethane is added dropwise. The solution is stirred for 10 minutes and then concentrated. The resulting Ac-His-Asp-Leu-Leu-Val-Gly-Ala-Pro-Leu-OH is filtered off; M⁺ 976.

### Example 4

Analogously to example 3 Ac-Gly-Arg-His-Asp-Leu-Leu-Val-Gly-Ala-Pro-Leu-OH (M⁺ 1189) is obtained.

The examples below relate to pharmaceutical formulations which contain the compounds of the formula I or their acid addition salts.

### Example A: Tablets

A mixture of 1 kg of H-Gly-Asp-Gly-Arg-His-Asp-Leu-OH, 10 kg of lactose, 6 kg of microcrystalline cellulose, 6 kg of potato starch, 1 kg of polyvinylpyrrolidone, 0,8 g of talc and 0.1 kg of magnesium stearate is pressed into tablets in the customary manner such that each tablet contains 10 mg of active compound.

### Example B: Coated tablets

Tablets are pressed analogously to Example A and are subsequently coated in the customary manner with a coating of sucrose, potato starch, talc, tragacanth and coloring substance.

### Example C: Capsules

Hard gelatine capsules are filled with 1 kg of H-Gly-Asp-Gly-Arg-His-Asp-Leu-OH in the customary manner such that each capsule contains 5 mg of active compound.

### Example D: Ampoules

A solution of 1 kg of H-Gly-Asp-Gly-Arg-His-Asp-Leu-OH in 30 l of 1,2-propanediol is subjected to sterile filtration, and ampoules are filled with the solution, lyophilized under sterile conditions and subjected to sterile sealing. Each ampoule contains 2 mg of active compound.

### Example E: Ointment

500 mg of H-Gly-Asp-Gly-Arg-His-Asp-Leu-OH is mixed with 99.5 g of petroleum jelly under aseptic conditions.

### Example F: Injection vials

A solution of 100 g of H-Gly-Asp-Gly-Arg-His-Asp-Leu-OH and 5 g of disodium hydrogenphosphate in 3l of doubly distilled water is adjusted to pH 6.5 with 2 N hydochloric acid, sterile filtered, filled into injection vials and lyophilized under sterile conditions, and the vials are closed in a sterile manner. Each injection vial contains 5 mg of active compound.

Pharmaceutical formulations which contain one of the other active compounds of the formula I and/or their physiologically acceptable acid addition salts can be obtained analogously.

The preceding examples can be repeated with similar success by substituting the generically or specifically described reactants and/or operating conditions of this invention for those used in the preceding examples.

From the foregoing description, one skilled in the art can easily ascertain the essential characteristics of this invention, and without departing from the spirit and scope thereof, can make various changes and modifications of the invention to adapt it to various usages and conditions.

## Claims

1. A compound of formula Ia-In
(Ia) H-Leu-Leu-Val-Gly-Ala-Pro-Leu-Tyr-OH;
(Ib) H-Asp-Leu-Leu-Val-Gly-Ala-Pro-Leu-Tyr-OH;
(Ic) H-His-Asp-Leu-Leu-Val-Gly-Ala-Pro-Leu-Tyr-OH;
(Id) H-Arg-His-Asp-Leu-Leu-Val-Gly-Ala-Pro-Leu-Tyr-OH;
(Ie) H-Gly-Arg-His-Asp-Leu-Leu-Val-Gly-Ala-Pro-Leu-Tyr-OH;
(If) H-Asp-Gly-Arg-His-Asp-Leu-Leu-Val-Gly-Ala-Pro-Leu-Tyr-OH;
(Ig) H-Gly-Asp-Gly-Arg-His-Asp-Leu-Leu-Val-Gly-Ala-Pro-Leu-Tyr-OH;
(Ih) H-Gly-Asp-Gly-Arg-His-Asp-Leu-OH;
(Ii) H-Gly-Asp-Gly-Arg-His-Asp-Leu-Leu-OH;
(Ij) H-Gly-Asp-Gly-Arg-His-Asp-Leu-Leu-Val-Gly-OH;
(Ik) H-Gly-Asp-Gly-Arg-His-Asp-Leu-Leu-Val-Gly-Ala-OH;
(Il) H-Gly-Asp-Gly-Arg-His-Asp-Leu-Leu-Val-Gly-Ala-Pro-Leu-OH;
(Im) Ac-His-Asp-Leu-Leu-Val-Gly-Ala-Pro-Leu-OH;
(In) Ac-Gly-Arg-His-Asp-Leu-Leu-Val-Gly-Ala-Pro-Leu-OH;
wherein Ac stands for acetyl, or a salt thereof.

2. A pharmaceutical composition comprising an amount of a compound of claim 1 effective to inhibit cell adhesion and a pharmaceutically acceptable carrier.

3. A composition according to claim 2, wherein the amount of said compound is 0.05-500 mg.

4. A composition according to claim 2, wherein the amount of said compound is 0.5-100 mg.

5. A method of treatment or prophylaxis of thrombosis, myocardial infarct, angina pectoris, apoplexy or tumors in a patient comprising administering to said patient a compound of claim 1.

6. A method of inhibiting adhesion of animal cells comprising administering a compound of claim 1.

7. A method according to claim 6, wherein said cells are somatic cells of animals.

8. A method according to claim 6, wherein said cells are cancel cells of mammals.

9. A method according to claim 5, wherein said compound is administered in a daily dosage of 0.1-2 mg per kg of body weight.

10. A method according to claim 6, wherein said compound is administered in a daily dosage of 0.1-2 mg per kg of body weight.
